# EUROPEAN PATENT SPECIFICATION

(11) **EP 2 309 870 B1**
(45) Date of publication and mention of the grant of the patent: **06.06.2012**
(21) Application number: 09745474.8
(22) Date of filing: 12.05.2009
(51) Int. Cl.: A23C 9/123, C12R 1/25, A61K 35/74

(54) **ISOLATED MICROORGANISM STRAIN LACTOBACILLUS PLANTARUM TENSIA DSM 21380 AS ANTIMICROBIAL AND ANTIHYPERTENSIVE PROBIOTIC, FOOD PRODUCT AND COMPOSITION COMPRISING SAID MICROORGANISM AND USE OF SAID MICROORGANISM FOR PREPARATION OF ANTIHYPERTENSIVE MEDICINE AND METHOD FOR SUPPRESSING PATHOGENS AND NON-STARTER LACTOBACILLI IN FOOD PRODUCT**
ISOLIERTER MIKROORGANISMENSTAMM LACTOBACILLUS PLANTARUM TENSIA DSM 21380 ALS ANTIMIKROBIELLES UND BLUTDRUCKSENKENDES PROBIOTISCHES NAHRUNGSMITTELPRODUKT, UND ZUSAMMENSETZUNG UMFASSEND DEN MIKROORGANISMUS, UND VERWENDUNG DES MIKROORGANISMUS FÜR DIE HERSTELLUNG VON BLUTDRUCKSENKENDEN ARZNEIMITTELN UND VERFAHREN ZUM SUPPRIMIEREN VON PATHOGENEN UND NICHT-STARTER-LACTOBACILLI IN EINEM NAHRUNGSMITTEL
SOUCHE DE MICROORGANISME ISOLÉE LACTOBACILLUS PLANTARUM TENSIA DSM 21380 EN TANT QUE PROBIOTIQUE ANTIMICROBIEN ET ANTIHYPERTENSIF, PRODUIT ALIMENTAIRE ET COMPOSITION COMPRENANT LEDIT MICROORGANISME ET UTILISATION DUDIT MICROORGANISME POUR LA PRÉPARATION D'UN MÉDICAMENT HYPERTENSIF ET PROCÉDÉ POUR SUPPRIMER DES PATHOGÈNES ET DES LACTOBACILLES NON UTILISÉS COMME LEVAIN DANS UN PRODUIT ALIMENTAIRE

(30) Priority: 13.05.2008 EE 200800026
(43) Date of publication of application: 20.04.2011
(73) Proprietor: Oü Tervisliku Piima Biotehnoloogiate Arenduskeskus, 51014 Tartu (EE)
(72) Inventor: SONGISEPP, Epp, EE50101 Tartu (EE); MIKELSAAR, Marika, EE51005 Tartu (EE); RÄTSEP, Merle, EE50109 Tartu (EE); ZILMER, Mihkel, EE 50406 Tartu (EE); HÜTT, Pirje, EE50110 Tartu (EE); UTT, Meeme, EE51707 Tartu (EE); ZILMER, Kersti, EE50406 Tartu (EE); ÜKSTI, Janne, EE51004 Tartu (EE); KÖLJALG, Siiri, EE50404 Tartu (EE)
(74) Representative: Kahu, Sirje
(86) International application number: PCT/EE2009/000005
(87) International publication number: WO 2009/138091

(56) References cited:
- MIKELSAAR MARIKA ET AL: "Lactobacillus fermentum ME-3-an antimicrobial and antioxidative probiotic" MICROBIAL ECOLOGY IN HEALTH AND DISEASE, vol. 21, no. 1, 2009, pages 1-27, XP002550026 ISSN: 0891-060X
- SONGISEPP EPP ET AL: "Lactobacillus plantarum tensia (DSM 21380) and L. plantarum inducia (DSM 21379) as novel probiotics" INTERNET ARTICLE, [Online] 11 November 2008 (2008-11-11), XP002550320 Mexico Retrieved from the Internet: URL:https://www.etis.ee/ShowFile.aspx?File VID=23482> [retrieved on 2009-10-14]
- KULLISAAR TIIU ET AL: "Two antioxidative lactobacilli strains as promising probiotics" INTERNATIONAL JOURNAL OF FOOD MICROBIOLOGY, vol. 72, no. 3, 1 January 2002 (2002-01-01), pages 215-224, XP002214344 ELSEVIER SCIENCE PUBLISHERS, AMSTERDAM, NL ISSN: 0168-1605 cited in the application
- DATABASE EPODOC EUROPEAN PATENT OFFICE, THE HAGUE, NL; PA: UNIV SOUTHERN YANGTZE (CN): "Lactobacillus plantarum CW006 with antihypertensive function" XP002550312 & CN 1 844 363 A (UNIV SOUTHERN YANGTZE [CN]) 11 October 2006 (2006-10-11)
- JAUHIAINEN T ET AL: "Lactobacillus helveticus Fermented Milk Lowers Blood Pressure in Hypertensive Subjects in 24-h Ambulatory Blood Pressure Measurement" AMERICAN JOURNAL OF HYPERTENSION, vol. 18, no. 12, 1 December 2005 (2005-12-01), pages 1600-1605, XP025334974 ELSEVIER ISSN: 0895-7061 [retrieved on 2005-12-01]
- DE VRIES M C ET AL: "Lactobacillus plantarum-survival, functional and potential probiotic properties in the human intestinal tract" INTERNATIONAL DAIRY JOURNAL, vol. 16, no. 9, 1 September 2006 (2006-09-01), pages 1018-1028, XP024963305 ELSEVIER APPLIED SCIENCE, BARKING, GB ISSN: 0958-6946 [retrieved on 2006-09-01]

### SEQUENCE LISTING

<110> Bio-Competence Centre of Healthy Dairy Products OÜ
<120> Isolated microorganism strain *Lactobacillus plantarum* Tensia DSM 21380 as antimicrobial and antihypertensive probiotic, food product and composition comprising said microorganism and use of said microorganism for preparation of antihypertensive medicine and method for suppressing pathogens and nonstarter lactobacilli in food product
<160> 8
<210> 1
   <211> 17
   <212> DNA
   *<213> Lactobacillus plantarum*
   <223> designation E1F
<400> 1
   ATCGGCAGGCCCAACAG
<210> 2
   <211> 21
   <212> DNA
   *<213> Lactobacillus plantarum*
   <223> designation E1R
<400> 2
   GCGTGACCGTGAATTAAATGC
<210> 3
   <211> 21
   <212> DNA
   *<213> Lactobacillus plantarum*
   <223> designation F1F
<400> 3
   GACAGCGCTAATGACCCAATC
<210> 4
   <211> 21
   <212> DNA
   *<213> Lactobacillus plantarum*
   <223> designation F1R
<400> 4
   GCGTGACCGTGAATTAAATGC
<210> 5
   <211> 19
   <212> DNA
   *<213> Lactobacillus plantarum*
   <223> designation J1F
<400>5
   CCCAACAACCCCGTTCAAC
<210> 6
   <211> 26
   <212> DNA
   *<213> Lactobacillus plantarum*
   <223> designation J1R
<400> 6
   TGCTACAGTAAAGGGAGAGGTATGAC
<210> 7
   <211> 26
   <212> DNA
   *<213> Lactobacillus plantarum*
   <223> designation K2F
<400> 7
   ACTTATTATAATCCCTTGAACCACCAA
<210> 8
   <211> 25
   <212> DNA
   *<213> Lactobacillus plantarum*
   <223> designation K2R
<400> 8
   GCTGACGCTGAAAAGAATATTTCTG

## Description

Isolated microorganism strain *Lactobacillus plantarum* Tensia DSM 21380 as antimicrobial and antihypertensive probiotic, food product and composition comprising said microorganism and use of said microorganism for preparation of antihypertensive medicine and method for suppressing pathogens and non-starter lactobacilli in food product

### TECHNICAL FIELD

The present invention relates to a novel microorganism and its applications. The invention belongs to the field of biotechnology and is used in food industry and medicine. More specifically the object of the present invention is antimicrobial and antihypertensive probiotic microorganism strain *Lactobacillus plantarum* Tensia DSM 21380. The invention relates to its use in the composition of a functional food e.g. cheese and for preparation of antihypertensive medicine.

### BACKGROUND ART

Lactobacilli used for production of fermented foods have been consumed for centuries. During the past decades lactobacilli have widely been used as probiotics. Probiotics are live microorganisms of human origin, which, when administered in adequate amounts, confer a health benefit on the host. Commonly probiotics are used for creating functional foods. Food can be regarded as functional, if beyond adequate nutritional components it contains some natural additives (pre- or probiotics), which beneficially affect one or more target functions in the body, either improving the state of health and well-being and/or reducing disease risk.

Probiotic products may be conventional foods (yoghurt, quark, curds) or dietary supplements (freeze-dried microbial cultures)

In European countries several probiotics supplied with health claims are marketed

### Antimicrobial probiotics

Antimicrobial probiotics against opportunistic pathogens and food-borne pathogens e.g. Salmonella have been described in patent applications. In WO2008/039531 (Little Columet Holdings KKC) the application of strains of *Lactobacillus acidophilus, L. bulgaricus, L. casei, L. paracasei, L. fermentum, L. plantanum, L. rhamnosus, L. salivarius, Bifidobacterium bifidum, B. infantis, B. animalis subsp. lactis, B. longum, Streptococcus thermophilus, Enterococcus faecalis* and *E. faecium* is described. The oral administration of these strains helps to suppress *Campylobacter jejuni, E. coli, S. aureus, Vibrio cholera, Bacteroides sp, Clostridium sp, Klebsiella sp, Listeria sp, Proteus sp, Salmonella sp, Shigella sp* and *Veillonella sp* in gastrointestinal tract.

Patent application WO2008/016214 (Bioneer Corporation) describes a *Lactobacillus gasseri* strain BNR17 with antimicrobial activity against *E*. *coli, S. aureus, S. typhimurium, B. cereus, L. monocytogenes* and *P. mirabilis.*

Persons skilled in the art are aware of the problem caused in cheese by non starter lactobacilli (NSLAB), which appear during cheese ripening. These microbes can cause unwished proteolysis, appearance of various undesirable flavour compounds and loss of commercial quality of cheese.

Affecting the counts of NSLAB during cheese maturation process with pH regulators, antioxidants and preservatives (NaCl) has failed. The addition of different antimicrobial compounds producing (lactic acid and acetic acid, H₂O₂) antagonistic microbes incl. probiotic lactobacilli into cheese has also been ineffective. H₂O₂ producing probiotic lactobacilli have been described by several authors (Ouwehand, A.C. Westerlund, Antimicrobial components of lactic acid bacteria. In: Lactic acid bacteria: microbiological and functional aspects. Eds. Salminen, S; Wright, A., .Ouwehand, A.C. 2004, pp 375-395, New York, Marcel Dekker; Hütt, P., Shchepetova, J., Lõivukene, K., Kullisaar, T., Mikelsaar, M. Antagonistic activity of probiotic lactobacilli and bifidobacteria against entero- and uropathogens. J. Appl. Microbiol., 2006, 100, 1324-1332).

Lactobacilli of human and plant origin adapt poorly in cheese environment rich in protein and fat and poor in carbohydrates, which is atypical for them. Therefore, only few probiotic lactobacilli comprising cheeses are available (Gardiner, G, Ross, R. P, Collins, J. H, Fitzgerald, G, and Stanton. C. Development of a probiotic cheddar cheese containing human derived Lactobacillus paracasei strains, Appl. Environ. Microbiol., 1998, 64,. 6: 2192-2199; Ross, R P, Fitzgerald, G F, Collins, J K, O'sullivan, G C, Stanton, C G. Process for the manufacture of probiotic cheese, US Patent 6872411, Patent application WO 99/62348 AI), in which the added probiotic bacteria sustain their ability to multiply - a prerequisite for the expression of their physiological- biochemical properties. However, in cheese, that reportedly contained probiotic LGG, the presence of the strain was undetectable (Coeuret V Probiotic lactobacilli from feed or cheese origin: enumeration, identification, properties and specific use. Thesis de Doctoral de Univ. De Caen Basse Normandie, France 2004; Coeuret V, Dubernet S, Bernardeau M, Gueguen M, Vernon Jp. Isolation, characterization and identification of lactobacilli focusing mainly on cheeses and other dairy products. Lait, 2003, 269-306).

The incorporation of probiotic lactobacilli with strong antagonistic activity against pathogens into food product and into human organism helps to avoid food-borne infections. The ability of live lactobacilli (LB) to produce bacteriocins is an essential prerequisite for suppression and elimination of related gram-positive as well as gram-negative bacteria, including food-borne pathogens. Bacteriocins help to improve quality of a food product and prolong its shelf-life. Natural synthesis of aforementioned antimicrobial substances by LB in food product or application of synthetic analogues in food products is essential in the case of food products low production temperature and NaCl concentrations e.g. spread cheeses and in countries with warmer climate (Lindgren and Dobrogosz Antimicrobial compounds, including bacteriocins produced by LAB increase the self life of the product, FEMS Microbiol Rew 1990, 87 149-164).

*L. plantarum* and *L. fermentum* strains, isolated from boza have a property to produce bacteriocins that inhibit gram-positive microbes. These bacteriocins are either proteins or peptides with antimicrobial properties (Mollendorff J.W., Todorov, S. D., Dicks, L:.M:.T. Comparison of Bactetiocins Produced by Lactic-Acid Bacteria Isolated from Boza, a Cereal-Based Fermented Beverage from the Balkan Peninsula Current Microbiol. 2006, 53; 209-216). Production of plautaricins is typical to several *L. plantarum* strains. Sub-class IIa includes food-borne pathogens inhibiting bacteriocins, sub-class IIb harbours dipeptide bakteriocins of wide activity range: plantaricin EF and plantaricin JK, also plantaricin S (Maldonado A., Ruiz-Barba JL, Floriano B, Jimenez-Diaz R., Int J Food Microbiol. 2002, 77(1-2):117-124. "The locus responsible for production of plantaricin S, a class IIb bacteriocin, produced by L. plantarum LPCO10, is widely distributed among wild-type L. plantarum strains isolated from olive fermentation"). Well-known is the application of plantaricin S producing *L. plantarum* strains LP RJ1 and LPRJL2 in the vegetable fermentation process (WO02/05665, WO0060948, Consejo Superior de Investigaciones Cientificas). The latter belongs to similar group of antimicrobial compounds with colicins, which attack mostly members of the same species and genus (Heinaru A, Tallmeister E. Colicin susceptibility of shigellas and coli bacteria related to episomal resistance and colicinogenicity or dissociation into S-R forms. Geneetika, 1971, 7, 5, 113-122 (in Russian). Therefore they are promising substances for suppression of undesirable nonstarter lactobacilli.

Production of plantaricins is regulated with several genes, some of which occur occasionally, some regularly. Therefore the phenotypical differences in plantaricin production occur. Genomic locus, associated with bacteriocin production has been described in *Lactobacillus plantarum* strain 11. Two of these operons of genes where termed *plnEFI* and *plnJKLR,* each having gene pair plnEF and plnJK encoding two small cationic bacteriocin-like peptides with double-glycine-type leader sequences. (Diep, D. B., Havarstein, L. S. & Nes, I. F. Characterization of the locus responsible for the bacteriocin production in Lactobacillus plantarum C11. J Bacteriol 1996, 178, 4472-4483). No disclosure is available concerning *L. plantarum* strains that can suppress NSLAB and. possess full set of plantaricin encoding genes EF and JK.

### Antihypertensive probiotics

More people suffer from metabolic syndrome which symptoms include overweight, obesity, and raise of blood pressure, blood glucose content and several other risk markers of atherosclerosis. Foods rich in fat (incl. cheese) are not problem-free, as these foods can increase the risk of developing atherosclerosis, inflammation, type II diabetes and/or lipid peroxidation (Raff M., Tholstrup T., Basu S., Nonboe P., Sorensen MT, Straarup EM. 138.509-514. A diet rich in conjugated linoleic acid and butter increases lipid peroxidation but does not affect atherosclerotic, inflammatory, or diabetic risk markers in healthy young men (American Society for Nutrition J. Nutr. 2008, 138:509-514).

Conjugated linoleic acid (CLA) refers to two naturally occurring isomers of 18-charbohyrdate linoleic acid (LA, *cis*-9, *cis*-12-18:2). CLA forms in the process of natural biohydrogenisation and oxidation. CLA isomers are formed during biohydrogenation of linoleic acid in the rumen and also through conversion of vaccenic acid in the mammary gland. In optimal dosages the conjugated linoleic acid (CLA) isomers have high health amelioration potential: antimicrobial, antitumorigenic, antidiabetic, anti-obesity and anti-allergic. Antihypertemsive effect has also been demonstrated by several research groups (Inoue K., Okada F., Ito R., Kato S., Sasaki S., Nakajima S., Uno A., Saijo Y., Sata F., Yoshimura Y., Kishi R. and Nakazawa H., Perfluorooctane sulfonate (PFOS) and related perfluorinated compounds in human maternal and cord blood samples: assessment of PFOS exposure in a susceptible population during pregnancy, Environ. Health Perspect. 112 (2004), pp. 1204-1207).

Supplementation of CLA in high amounts with food is, however, problematic. It has been demonstrated, that consumption of 5g CLA daily leads to the increase of lipid peroxidation (LPO), expressed in the elevated concentration (83%) of 8-isoprostaglandine F₂ (Raff M., Tholstrup T., Basu S., Nonboe P., Sorensen MT, Straarup EM. 138.509-514. A diet rich in conjugated linoleic acid and butter increases lipid peroxidation but does not affect atherosclerotic, inflammatory, or diabetic risk markers in healthy young men American Society for Nutrition J. Nutr. 2008, 138:509-514).

Best solution is the usage of lactobacillus strain possessing both the ability of moderate CLA production and physiologically relevant antioxidative capacity. Incorporation of physiologically relevant antioxidative LAB into food could control the increase of LPO and elevates the bio-suitability of fat-rich foods e.g. cheese. *Lactobacillus fermentum* ME-3 described in Tartu University patent EE04580 and in research articles possesses remarkable physiological antioxidative activity (Kullisaar T, Zilmer M, Mikelsaar M, Vihalemm T, Annuk H, Kairane C, Kilk A. Two antioxidative lactobacilli strains as promising probiotics. Int. J. Food Microbiol., 2002, 72, 215-224; Kullisaar, T., Songisepp, E., Mikelsaar, M., Zilmer, K., Vihalemm, T., Zilmer, M. Antioxidative probiotic fermented goats' milk decreases oxidative stress-mediated atherogenicity in human subjects. Br. J. Nutr., 2003, 90, 449-456; Truusalu, K. Naaber, P., Kullisaar, T., Tamm, H., Mikelsaar, R-H., Zilmer, K., Rehema, A., Zilmer, M., Mikelsaar, M. The influence of antibacterial and antioxidative probiotic lactobacilli on gut mucosa in a mouse model of Salmonella infection. Microbial Ecology in Health and Disease 2004, 16:4, 180-187).

Concentration of polyamines in human blood and exudation with urine fluctuate according to the consumption of food with different polyamines concentrations, but also in connection with endogenous polyamines producers in human intestinal microflora like *E. coli* and certain anaerobes (Marino M. Maifreni M, Moret S., Rondinini G. The capacity of Enterobacteriaceae sp. to produce biogenic amines in cheese. Letters of Microbiology, 2000, 31, 169-173). Several food products contain polyamines in relatively high concentrations e.g. oranges contain considerable amounts of putrescine 1330 µg/100g (Larqué E., Sabater-Molina M., Zamora S. Biological significance of dietary polyamines. Nutrition, 2007, 23, 87-95.). Lactobacilli strains, which are able to produce moderate concentrations of CLA and polyamines and at the same time possess physiologically relevant antioxidativity have not been described.

Blood pressure is regulated by compounds like nitrogen monooxide (NO), lipid peroxidation (LPO), oxidized low density lipoproteins (ox-LDL) and components of glutathione red-ox system (GSSG/GSH), which modulate blood vessel stiffness and affect their vasoconstriction.

Moreover, the raise of LPO, ox-LDL, GSSG/GSH levels is one risk factor of atherosclerosis. Metabolism of acetylated spermidine provides additional possibilities for vasodilatory (blood pressure lowering) effect through acetylated form and polyamin ordinary forms (Myung CS, Blankenship JW, Meerdink DJ. A mechanism of vasodilatory action of polyamines and acetylpolyamines: possible involvement of their Ca2+ antagonistic properties, J Pharm Pharmacol. 2000, 52:695-707).

Some peptides, harboured in milk possess blood pressure lowering ability through inhibition of angiotensine converting enzyme I (ACE) (Meisel, H. & Bocklemann, W. Bioactive peptides encrypted in milk proteins: proteolytic activation and tropho-functional properties. In: Proceedings of the sixth symposium on lactic acid bacteria: genetics, metabolism and applications. 19-23 September. Veldhoven (W. N. Konings, O. P. Kuipers, and J. Huis in't Veld., eds.) Kluver Academic Publishers, the Netherlands. 1999, pp. 207-215 (1999)).

Nitric monoxide NO belongs to bioactive compounds, having several beneficial effects like antimicrobial and anti-inflammatory activity and blood pressure lowering property, (Janeway, CA, Travers, P, Walport, M, Shlomchik, MJ. 2005. Immunobiology: The Immune System in Health and Disease. New York, NY: Garland Science Publishing). NO affects blood pressure though different pathways like relaxation of smooth muscles of blood vessels, ACE inhibition or promotion of endogenous protection mechanisms (*preconditioning*) (Jones SP, Bolli R The ubiquitous role of nitric oxide in cardioprotection. J Mol Cell Cardiol 2006; 40: 16-23).

Today only a few disclosures are available concerning probiotic microorganisms, which produce NO endogenously or promote NO endogenous production. Lactic acid bacteria with promote endogenous NO production are described in patent applications of Valio Ltd Nos EE00200230A and EE200200231. Patent application EE00200230A discloses lactobacillus strain *L. helveticus* LBK-16H, DSM 13137 with photolytic activity and which produces di- and tri-peptides. Those di- and tri-peptides derived form casein due to lactic fermentation are concentrated by nano-filtration and when applied into food products incl. fermented milk products, an antihypertensive peptides containing food has been created. Patent application EE200200231 discloses, that strain *L. helveticus* LBK-16H, DSM 13137 in addition to di- and tri-peptide production ability is also able to induce NO production in two cell lines (mice and human enterocytes). The strain assists blood pressure lowering through the production of peptides and NO. The authors note, that the bacterial cells were not the only NO producers (page 13, lines 8-9).

USA patent US7183108 (Compagnie Gervais Danone, 2007) discloses, that *Lactobacillus casei* possesses anti-inflammatory ability due to the increase of NO production (NO is produced by proinflammatory cytokine activated enterocytes), and *vice versa -* the bacterial NO production is down regulated when enterocytes are already activated by proinflammatory cytokines and bacterial lipopolysaccharides.

Korhonen *et al.* 2001 (Korhonen K, Reijonen TM, Remes K, Malmström K, Klaukka T, Korppi M. Reasons for and costs of hospitalization for paediatric asthma: A prospective 1-year follow-up in a population-based setting. Pediatr Allergy Immunol 2001:12:331-338) showed, that *Lactobacillus rhamnosus* GG could stimulate NO production by intestinal epithelial cells or by induction of proinflammatory cytocines. These authors pointed out the possible desirable connection of LGG and NO production.

European patent EP0951290 (Laboratories Standa S.A., 2002) discloses NO-producing propionibacteria applied into the composition for production of NO in gastrointestinal tract, presumably in enterocytes. Ibidem a strain of *Lactobacillus farciminis* is mentioned as a negative example (the producer of low NO amounts). USA patent US 7294337 (Institut National de la Recherche Agronomique, 2007) however demonstrates that *L*. *farciminis* is able to produce NO in amounts sufficient for anti-inflammatory effect and suppression of pain after peritoneum stretch.

It has been disclosed that *L. fermentum* strain LF1 was able to produce NO aerobically in MRS broth without the presence of human cell culture. The *L. plantarum* strain DSM9843 (LP2), tested by same authors, was not able to act similarly, opposite to our strain *L. plantarum* Tensia DSM 21380 (J.Xu, W. Verstraete. Evaluation of nitric oxide production by lactobacilli. Appl. Microbiol. Biotechnol., 2001, 56:504-507). Incorporation into human organism a strain as a probiotic with aforementioned properties allows controlling the blood pressure due to the NO production ability of the strain and the property of NO to affect blood pressure pathways, as described above.

Besides the poor survival in food products, the selection of probiotic bacteria faces the problem of poor survival of strains thought the passage of the gastrointestinal environment. Therefore, such probiotic lactobacilli are needed, which are able to survive both in the upper as well as the lower parts of gastrointestinal tract. The international patent application WO 91/05850 (Tartu University et al., 1989) describes the strain *L*. *plantarum* 38, as beneficial for adjusting colonic microflora in the case of bacterial dysbiosis. European patent EP0554418 B1 (Probi AB, 1998) discloses the good colonization properties of the strain *L. plantarum* 299 (DSM 6595) in the human intestine and the application of the strain with oatmeal-drink for surgical patients against enteric infection. International patent application WO2007/003917 A1 (Matforsk AS et al, 2006) describes *Lactobacillus plantarum* DSM 16997 (DSM 17320) strains and a strain of *L. pentosus* (DSM17321) as starter microbes in meat products. According to authors, these strains can function as probiotics balancing human intestinal microflora through suppression of harmful bacteria and also commensial *Escherichia coli.* To prove these claims, the results of *in vitro* experiments are presented. However, no proof of pathogen suppression abilities *in vivo,* (i.e. on animal model) is given. Besides, the suppression of non-starter lactobacilli (NSLAB) is not demonstrated, although survival of NSLAB in sausage environment is described at low pH values. Without proof the probiotic strains are attributed immunostimulatory and sepsis-lowering properties after surgical interventions and the improvement of patients' well-being through the administration of aforementioned probiotic strains.

Patent EE04097 B1 (Probi AB, 2003) discloses the application of *Lactobacillus plantarum* 299v (DSM 9843) for treatment of urinary infections, whereas the strain is able to adhere to the human colonic epithelium because of its mannose-specific adhesins and to compete with harmful urinary tract pathogens for adhesion sites. According to REA (restriction enzyme type) analysis all aforementioned strains differ from each other and from *Lactobacillus plantarum* Tensia DSM 21380 for the clearly distinct chromosome profile.

### DISCLOSURE OF THE INVENTION

The present invention relates to a novel isolated microorganism strain *Lactobacillus plantarum* Tensia DSM 21380 as antimicrobial and antihypertensive probiotic, food products (dairy products, e.g. fermented products or cheese) and composition comprising said microorganism and use of said microorganism for production of antihypertensive medicine. The invention also relates to method for suppressing pathogens and non-starter lactobacilli in food product and method for extension of shelf life of food product by the means of *Lactobacillus plantarum* Tensia DSM 21380.

*Lactobacillus plantarum* Tensia DSM 21380 as antimicrobial probiotic produces antimicrobial substances - lactic acid, acetic acid, CLA, NO, H₂O₂ and possesses plantaricin II b class gene pairs EF and JK encoding antimicrobial compound. With its metabolites *Lactobacillus plantarum* DSM 21380 suppresses *in vitro* both non-starter lactobacilli and enteric-pathogens. The strain is able to germinate and predominate among lactobacilli at low temperatures and in the condition of carbohydrate starvation (e.g. cheese).

In addition to antimicrobial activity *Lactobacillus plantarum* Tensia DSM 21380 is also an antihypertensive and antioxidative probiotic due to the production of NO, CLA and antioxidativity, produces *in vitro* small amounts of polyamines tyramine and putrescine and prevails in the intestinal microflora of volunteers after 3-week consumption of *Lactobacillus plantarum* Tensia DSM 21380 comprising cheese. The good colonization ability of *Lactobacillus plantarum* Tensia DSM 21380 in the human intestine was detectable in fecal samples both by DGGE profile and by methods of classical bacteriology. *Lactobacillus plantarum* Tensia DSM 21380 significantly activates the metabolism of putrescine and acetylated spermidine in the human body, accompanied by lowering both systolic and diastolic blood pressure.

### DESCRIPTION OF THE STRAIN

The object of the invention, the microorganism strain *Lactobacillus plantarum* Tensia DSM 21380 was isolated from a fecal sample of a healthy child during a comparative study of the microflora of Estonian and Swedish children. The microorganism *L plantarum* Tensia DSM 21380 was isolated, by seeding the dilutions of the faeces of a healthy one-year old Estonian child (10⁻² - 10⁻⁷ in phosphate buffer with 0.04% thioglycol acid; pH 7.2). The dilutions were seeded on freshly prepared MRS agar medium (Oxoid) and cultivated at 37°C microearobically. The strain, which is the object of the invention, was isolated according to the characteristic morphology of colonies and cell to *Lactobacillus sp.* A provisional and more precise identification followed, as described below.

The strain, which is the object of the investigation, was isolated according to the characteristic morphology of colonies and cell to *Lactobacillus sp.* A provisional and more precise identification followed as described below.

The fact that the microbial strain *Lactobacillus plantarum* Tensia DSM 21380 originates from the intestinal tract of a healthy child, proves its GRAS (generally recognized as safe) status i.e. that this strain of microorganism is harmless for human organism and is suitable for oral application.

The microorganism *Lactobacillus plantarum* strain Tensia was deposited in Deutsche Sammlung von Mikroorganismen und Zellkulturen GmbH under the registration number DSM 21380 at 16.04.2008.

**Cultural - morphological characteristics** were detected after cultivation on MRS agar and in MRS broth (OXOID). Cells of *Lactobacillus plantarum* Tensia DSM 21380 are Gram-positive, non-spore-forming rods of regular shape, occurring singly, in pairs or in short chains.

### Physiological-biochemical characteristics

The MRS broth was suitable for cultivation of the strain during 24- 48 h in microaerobic environment, after which homogenous turbid growth occurred in the broth. Colonies on MRS agar plates after 48h of growth at 37°C in microaerobic conditions (atmosphere CO₂/O₂/N₂: 10/5/85) are round, 2-2.5 mm of diameter, sooth, entire, convex and white.

The optimal growth temperature is 37°C; it multiplies also at 15°C and 45°C. The optimal pH of the growth environment is 6.5.

*Lactobacillus plantarum* strain Tensia DSM 21380 is catalase and oxidase negative, facultatively heterofermentative, no gas is produced from glucose and no arginine hydrolysis.

Strain *Lactobacillus-plantarum* Tensia DSM 21380 was identified on the basis of biochemical activity with API 50CHL System (bioMérieux, France) kit as *Lactobacillus plantarum* (Coincidence with the type strain: Excellent, ID %-99.9, T index -0.86).

The comparison with the reference strain *Lactobacillus plantarum* ATCC 14917 confirmed the preliminary identification by API 50CHL.

Carbohydrate utilization profile of *Lactobacillus plantarum* Tensia DSM 21380 according to API 50 CHL is as follows. Positive reaction for ribose, L- arabinose, galactose, D-glucose D-fructose, D- mannose, mannitol, α methyl-D-mannoside, α methyl-D-glucoside, N acetyl-glucosamine, amygdalin, arbutine, esculine, salitsin, cellobiose, maltose, lactose, melibiose, D-arabinose, sachharose, trehalose, melezitose, D-raffinose, β-gentiobiose, D-turanose, gluconate, weak reaction for starch.

According to API ZYM test-kit (bioMérieux, France), *Lactobacillus plantarum* Tensia DSM 21380 possesses leucine arylamidase, acid phosphatase, α-glucosidase, β - glucosidase and acetoin activity. Weak reaction for valine arylamidase, naphthol-AS-BI-phosphohydrolase, β-galactosidase, cystine arylamidase, esterase (C4), esterase (C8), N-acetyl- β-glucosaminidase was detected.

The molecular identification of *Lactobacillus plantarum* Tensia DSM 21380 was confirmed by Internal-Transcribed Spacer Polymerase Chain Reaction (ITS-PCR in comparison with the reference strain *Lactobacillus plantarum* ATCC 14917 (Fig.1).

**Method.** Strain identification was confirmed by (ITS-PCR) Internal-Transcribed Spacer Polymerase Chain Reaction in comparison with the reference strain *Lactobacillus plantarum* ATCC 14917.

The DNA extraction from *Lactobacillus* isolates was performed using lysozyme (Serva, Sweden; 20 mg/ml), mutanolysin (Sigma; 0.5 mg/ml) and proteinase K solutions (Fermentas, Lithuanian; 14.6 mg/ml).

The DNA amplification was performed in 1x*Taq* polymerase buffer, containing 1.5U *Taq* polymerase (Fermentas, Lithuania), 0.5 µM of each primer (16S-1500F and 23S-32R;

DNA Technology AS), 200 µM deoxynucleoside triphosphates (Amersham Pharmacia Biotech, Germany), 2 mM MgCl₂ and 2 µl of DNA under investigation.

Subsequently, the PCR product was restricted using a *Taq* I restriction enzyme (Fermentas, Lithuania). PCR products were separated by on a 2% agarose gel in 1xTBE buffer; voltage 100 V). The banding patterns were visualized in UV light and compared with reference stain *L. plantarum* ATCC 14917.

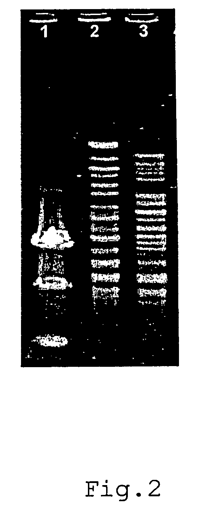

*Lactobacillus plantarum* Tensia DSM 21380 molecular fingerprints were compared with reference *L. plantarum* stain, using pulsfield-gel-electrophoresis (PFGE) (Fig. 2).

**Method.** For pulsfield-gel-electrophoresis (PFGE) procedure the lactobacillus strains were grown in MRS broth at 37°C for 24 h. The cells were washed in SE buffer (75mM NaCl, 25mM EDTA, pH=7.4), density of the cell suspension was adjusted to 1.5 (OD₆₀₀). The DNA extraction from *Lactobacillus* isolates was performed in EC buffer (50mM EDTA (pH 8.5), 0.5% Na-laurylsarcosine, 0.2% Na-deocycholate 2mg/ml lysozyme, 10U mutanolysin), followed by 1mg/ml proteinase K solution (100 mM EDTA-1% sarcosyl-0.2% deoxycholate, pH 8.0) containing buffer. Lysed probes were washed in TE buffer and cut to 2 mm, extracted overnight with enzyme 50U Not I (Bio-Rad). The electrophoresis was carried out in CHEF-DR II (Bio-Rad) for 22h at 14°C. The banding patterns were visualized in UV light illuminator.

### Antibiotic resistance

**Method.** *Lactobacillus plantarum* Tensia DSM 21380 was tested using antibiotic strips of the E-test (AB Biodisk, Solna). The MIC (minimal inhibitory concentration) was determined according to European Commission (EUC) suggested epidemiological break-points.

**Table 1. Antibiotic resistance of Lactobacillus plantarum Tensia DSM 21380**

| Antibiotic | MIC* (µg/ml) | MIC* (µg/ml) | EUC antibiotic resistance MIC* breakpoint values (µg/ml) |
|---|---|---|---|
| | *L. plantarum* Tensia DSM 21380 | Control strain *L. plantarum* DSM 21379 | |
| Ampicillin | 0.25 | 0.19 | 4 |
| Gentamicin | 1.5 | 1 | 64 |
| Streptomycin | 16 | 6 | 64 |
| Erytromycin | 0.19 | 0.25 | 4 |
| Clindamycin | 0.032 | 0.016 | 2 |
| Tetracycline | 8 | 6 | 32 |
| Chloramphenicol | 2 | 2 | 8 |
| Cipro/ofloxacin | 32 | 32 | 4 |
| Quinopristin/dalfopristin | 1 | 1 | 4 |

| | | | |
|---|---|---|---|
| * minimal inhibitory concentration | | | |

*Lactobacillus plantarum* Tensia DSM 21380 did not harbor antibiotic resistance against most important antimicrobial preparations.

Somewhat higher MIC for ciprofloxacin was detected in normal range of wild strains described previously. Therefore, likely it is wild type stain (Vankerckhoven V, Huys G, Vancanneyt M, Vael C, KlareI, Romond M-B, Entenza J M, Moreillon P, D. Wind R, Knol J, Wiertz E, Pot B., Vaughan E. E, Kahhneter G, Goossens H. Biosafety assessment of probiotics used for human consumption: recommendations from the EU-PROSAFE project. Trends in Food Science & Technology 2008; 19: 102e114) and no horizontal transfer of antibiotic resistance genes of *Lactobacillus plantarum* Tensia DSM 21380 during the application of the strain as a probiotic could be predicted.

### Functional properties

### The profile of metabolites

**Method.** The profile of metabolites was determined by gas chromatographic method (Hewlett-Packard 6890) after incubation in microaerobic milieu for 24 h and 48h (Table *2). L. plantarum* strain was grown on MRS agar for 48h microaerobically (10% CO₂). A suspension (McFarland 4.0 turbidity standard, 10⁹CFU/ml) of lactobacillus culture was prepared in 0.9% NaCl solution. 1.0 ml of this suspension was transferred to 9.0 ml of MRS broth. The metabolite concentration (mmol/l,) was detected using the capillary column HP-INNOWax (15 m ×0.25 mm; 0.15 µm). The column temperature program was 60°C 1 min, 20°C/min 120°C 10 min; detector (FID) 350°C.

Electrochemical measurements of H₂O₂ were carried out with 24h old intact cells in 500µl of MRS broth with Apollo 4000 free radical analyzer (WPI, Berlin, Germany) and electrodes of type ISO-HPO2 and ISO-NOP.

ISO-HPO2 electrode signals were registered during 5-7 minutes. Mean signal strength was calculated. Each experimental point was measured in 4 independent parallels and each parallel was measured twice. H₂O₂ concentration was calculated according to the standard curves correlation with the strength of the electrodes signal.

**Table 2. Acetic acid, lactic acid and succinic acid concentration (mmol/l) of Lactobacillus plantarum Tensia DSM 21380 in MRS broth at microaerobic cultivation for 24 and 48h and H₂O₂ concentration (µg/ml) of intact cells**

| *L. plantarum* | Acetic acid (mmol/l) | | Lactic acid (mmol/l) | | Succinic acid (mmol/l) | | H₂O₂ (µg/ml) |
|---|---|---|---|---|---|---|---|
| | 24 h | 48 h | 24 h | 48 h | 24 h | 48 h | intact cells 24 h |
| Tensia DSM 21380 | 1.4 | 1.7 | 112.2 | 129.2 | 0.6 | 0.6 | 196.4 ± 128.8 |
| DSM 21379 | 2.1 | 2.4 | 133.3 | 186.6 | 0.6 | 0.6 | 288.9 ±175.8 |

*Lactobacillus plantarum* Tensia DSM 21380 produces substantial amounts of acetic acid, lactic acid and hydrogen peroxide. The concentration of the latter according to Apollo analyzer signals is however somewhat lower than that of the control strain.

### Antimicrobial activity against pathogens and non-starter lactobacilli

*Lactobacillus plantarum* Tensia DSM 21380 expresses *in vitro* on MRS agar medium antagonistic activity against non-starter lactobacilli and several enteric pathogens (Table 3). This property enables to use the strain for prolongation of shelf-life of food products.

**Table 3. Lactobacillus plantarum Tensia DSM 21380 antimicrobial activity against pathogens and non-starter lactobacilli on modified MRS agar medium (pathogen growth inhibition zone, mm)**

| **Pathogen** | **Growth inhibition zone (mm)** |
|---|---|
| Non-starter lactobacilli (NSLAB) | 8.6 ± 4.07 |
| *Listeria monocytogenes* | 25.1±1.7 |
| *Yersinia enterocolitica* | 13.5± 1.7 |
| *Salmonella enteritidis* | 25.2 ± 1.5 |
| *S. enterica* serovar Typhimurium | 22.8 ± 0.1 |
| *Shigella sonnei* | 25.1 ± 1.6 |
| *Escherichia coli* | 29.8 ± 3.7 |
| *Enterobacter sakazakii* | 18.5±3.6 1 |
| *Campylobacter jejuni* | 12.9± 5.2 |

*Lactobacillus plantarum* Tensia DSM 21380 antimicrobial activity *in vitro* in streak-line procedure (antimicrobial effect of metabolites) was highest against *E*. *coli,* followed by growth inhibition of *Salmonella* sp., *Shigella sp* and *Listeria sp.* The lowest antimicrobial activity of *Lactobacillus plantarum* Tensia DSM 21380 was against other lactobacilli (NSLAB).

### Lactobacillus plantarum Tensia DSM 21380 bacteriocin genes

**Method:** Detection of *Lactobacillus plantarum* Tensia DSM 21380 bacteriocin encoding genes based on the *Lactobacillus plantarum* WCFS1 genes *plnE, plnF plnJ* and *plnK.* These genes encode bacteriocin precursor peptides (*Lactobacillus plantarum* WCFS1 genome annotation - Kleerebezem et al 2003. Complete genome sequence of Lactobacillus plantarum WCFS1. Proc Natl Acad Sci U S A. 2003 Feb 18; 100 (4): 1990-5). The PCR primers were designed using Primer Express®. *Lactobacillus plantarum* WCFS1 annotated genome was downloaded from NCBI genome browser (http://www.ncbi.nlm.nih.gov/Genomes).

The designed primers were designated as E1F, E1R, F1F, F1R, J1F, J1R, K2F, K2R (look: Sequence listing) and there use in PCR-reaction gave positive signals for all aforementioned sequences. *L. plantarum* BAA-793 NCIMB 8826 was used as positive control and strain *L. plantarum* DSM 21379 as negative control.

**Table 4. Presence of amplified PCR products of genomic DNA bacteriocin encoding genes plnE, plnF, plnJ and plnK ofLactobacillus plantarum DSM 21380**

| | Genes | | | |
|---|---|---|---|---|
| | *plnE* | *plnF* | *plnJ* | *plnK* |
| *L. plantarum* BAA-793 (NCIMB 8826) | + | + | + | + |
| *L. plantarum* Tensia DSM 21380 | + | + | + | + |
| *L. plantarum* DSM 21379 | + | + | - | - |

The antagonistic activity of *L. plantarum* Tensia DSM 21380 among other antimicrobial compounds is also based on aforementioned gene products e.g. antimicrobial peptides. Peptides E & F and J & K should be expressed simultaneously to get max response via di-peptides.

### Production of conjugated linoleic acid (CLA) by L.plantarum Tensia DSM 21380

**Method.** Production of CLA was determined in MRS broth (de Mann-Rogosa-Sharpe, Oxoid, UK) and in skim milk by spectrophotometrical measurements.

| | |
|---|---|
| MRS broth | 39.9 mg/l (moderate) |
| Skim milk | 19.2 mg/l (moderate) |
| Cheese | 3.0±0.3 mg/g |

### Production of nitrogen mono-oxide (NO) by L. plantarum Tensia DSM 21380

**Method.** Nitrogen mono-oxide production measurements were carried out with 24h old intact cells in 500 µ of MRS broth with Apollo 4000 free radical analyzer (WPI, Berlin, Germany) and electrodes of type. ISO-NOP electrode signals were registered during 5-7 minutes. Mean signal strength was calculated. Each experimental point was measured in 4 independent parallels and each parallel was measured twice. NO concentration was calculated according to the standard curves correlation with the strength of the electrodes signal.

**Table 5. NO concentration (µM) produced by L. plantarum Tensia DSM 21380**

| **Strain number** | **NO concentrations (µM)** |
|---|---|
| *L. plantarum* Tensia DSM 21380 | 2.6 ± 0.8 |
| *L. plantarum* DSM 21379 | 2.7 ± 1.2 |
| *L. coprophilus* | 2.1 ± 1.1 |
| *L. plantarum* | 2.1 ± 0.9 |
| *L. paracasei ssp paracasei* strain no 1 | 1.3 ± 0.8 |
| *L. paracasei ssp paracasei* strain no 2 | 1.8 ±0.9 |
| *L. paracasei ssp paracasei* strain no 3 | 2.8 ± 1.6 |
| *L. buchneri* | 2.0 ±1.1 |

*L. plantarum* Tensia DSM 21380 was one of the best NO producers in comparison with 8 other *Lactobacillus sp* strains.

### Antioxidative activity of Lactobacillus plantarum Tensia DSM 21380

**Method**: For the detection of TAA and TAS of the microbial cells, the strain *L*. *plantarum* Tensia DSM 21380 was incubated in MRS broth (Oxoid, U.K.) for 24h at 37°C. Microbial cells were harvested by centrifugation (1500 RPM, during 10 min) at 4°C and the pellet was washed with isotonic saline (4°C) and suspended in 1.15% KCl (Sigma, USA). The density of the suspension was OD ₆₀₀ of 1.1 x 10⁹ bacterial cells ml⁻¹). Total antioxidative activity (TAA) was assessed by using the linolenic acid test (LA-test). (Kullisaar, T, Songisepp, Mikelsaar M, Zilmer, K, Vihalemm, T, Zilmer, M. British J of Nutrition. Antioxidant probiotic fermented milk decreases oxidative stress-mediated atherogenicity in human. 2003, 90, 2, 449-456) and total antioxidative status (TAS) was measured by commercial kit (TAS, Randox Laboratories Ltd., UK) (Table 6).

**Table 6. Total antioxidative activity (TAA) and total antioxidative status of (TAS) Lactobacillus plantarum Tensia DSM 21380**

| Strain | TAA (%) | TAS (mmol/l) |
|---|---|---|
| *L. plantarum* Tensia DSM 21380 | 22±5 | 0.05±0.02 |
| *L. plantarum* DSM 21379 | 26±1.2 | 0.13±0.04 |

The values of TAA and TAS of the strain *L. plantarum* Tensia DSM 21380 were somewhat lower than of the strain *L. plantarum* DSM 21379.

*L. plantarum* Tensia DSM 21380 parameters were lower in comparison with the values of the antioxidative strain *L. fermentum* ME-3 (DSM14241) (ME-3, TAA: 29+0.7; TAS: 0.16±0.03).

***In vitro* polyamines production of *Lactobacillus plantarum* Tensia DSM 21380 Method**. Microbial strains were suspended in physiological saline according to McFarland turbidity standard (10⁹ CFU/ml) and 0.5 ml of each strain suspension was seeded into decarboxylation medium (á 4.5 ml) and incubated at 37°C for 4 days (Bover-Cid et al., 1999).

For detection of BA 200 µl of medium was derivatized for GC analyze by modified method of Nakovichi (Nakovich, L. Analysis of biogenic amines by GC/FID and GC/MS. Thesis, Virginia polytechnic institute, USA. 2003).

GC analyses were carried out by gas chromatograph HP 6890 Series GC System, with capillary colonna HP-5 19091J-413 (30 m ×0.32 mm; 0.25 µm). The column temperature program 160°C 1 min, 20°C/min 280°C 15 min; and detector (FID) 300°C.

**Table 7. Concentration of polyamines in vitro in decarboxylation medium Arena, M. E., Manca de Nadra, M. C. Biogenic amine production by Lactobacillus. J. Applied Microbiology 2001, 90, 158-162.**

| Sample | Polyamines (µg/ml) and biogenic amines | | | | | | | | |
|---|---|---|---|---|---|---|---|---|---|
| | Arginine | | Glutamine | | Lysine | | Ornithine | | Histidine |
| | Putre scine | Cadav erine | Putre scine | Cadav erine | Putre scine | Cadav erine | Putres cine | Cadav erine | Hista mine |
| *L. plantarum* Tensia DSM 21380 | 0 | 0 | 0 | 0 | 0 | 0.3 | 0.5 | 0.6 | 0 |
| *L. plantarum* DSM 21379 | 0 | 0.4 | 1.2 | 0.5 | 0 | 0.4 | 1.9 | 0 | 0 |
| *E. coli* ATCC 700336 | 18.4 | 1.7 | 12.3 | 18.4 | 1.8 | 240 | 1599.3 | 3.5 | 105.1 |

*L. plantarum* Tensia DSM 21380 produces small amounts of polyamine putrescine from ornithine. From biogenic amines, traces of cadaverine were detected. No production of histamine.

### DESCRIPTION OF THE DRAWINGS

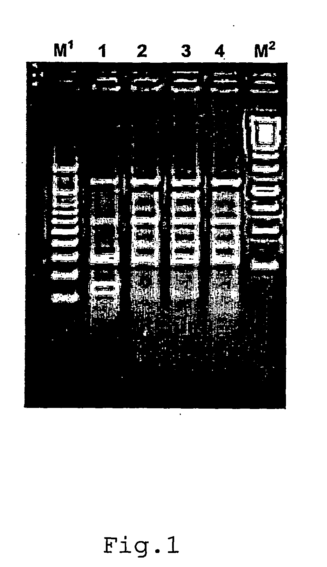

Fig.1. *Lactobacillus plantarum* Tensia DSM 21380 molecular identification by ITS-PCR
*1. L. plantarum* DSM 21379
*2. L. plantarum* Tensia DSM 21380
*3. L. plantarum* CRL 972 (ATCC 14917)
M- 100bp marker (Fermentas)

Fig.2. *L. plantarum* Tensia DSM 21380 molecular fingerprints in comparison with *L*. *plantarum* control strain (Pulse field-gel-electrophoresis profile, PFGE).
1. Lambda Ladder PFG Marker (New England Bio Labs *Inc.)*
*2. L. plantarum* Tensia DSM 21380
*3. L. plantarum* DSM 21379 (control)

Fig.3. *L. plantarum* Tensia DSM 21380 presence in cheese by DGGE method DGGE gel-electrophoresis form test-cheeses (cheeses with probiotic additive, control cheese, cheese with prebiotic additive).

| | | | |
|---|---|---|---|
| 1. | Cheese nr 529 | 9. | Cheese nr 9-2 |
| 2. | Control cheese | 10. | *L. plantarum* DSM 21379 |
| 3. | Cheese nr 5 | 11. | *L. plantarum* Tensia DSM 21380 cheese |
| *4.* | *L. plantarum* strain no 1 | 12. | *L. plantarum* Tensia DSM 21380 pure culture |
| 5. | Cheese nr 9-1 | 13. | Cheese nr 4 |
| *6.* | *L. plantarum* DSM 21379 | 14. | Cheese nr 12 |
| 7. | Cheese nr 19 | 15. | *L. paracasei* strain no 1 |
| *8.* | *L. plantarum* strain no 1 | 16. | *L. plantarum* DSM 21379 |

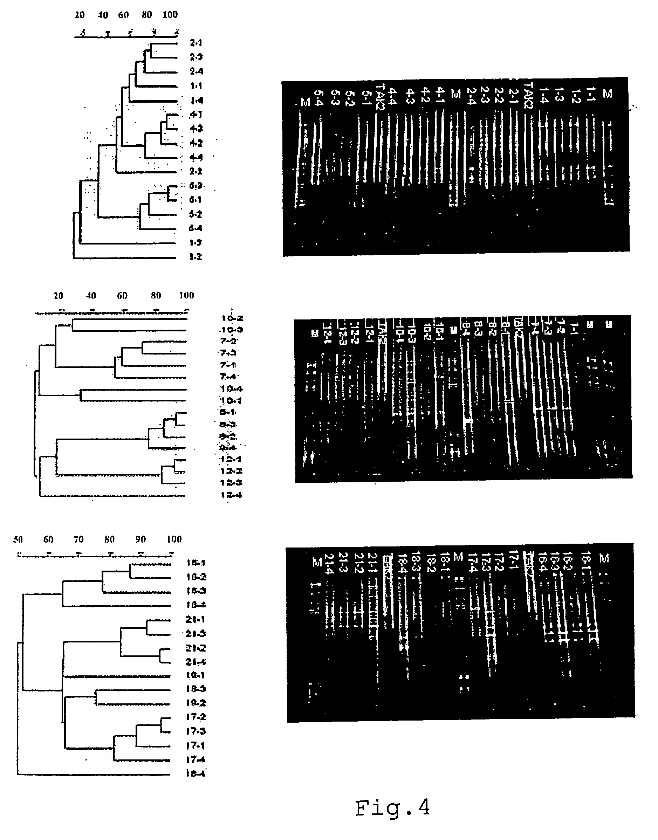

Fig.4. Lactobacillus species by Pearson UPMAG cluster analyses in *L. plantarum* Tensia DSM 21380 group.

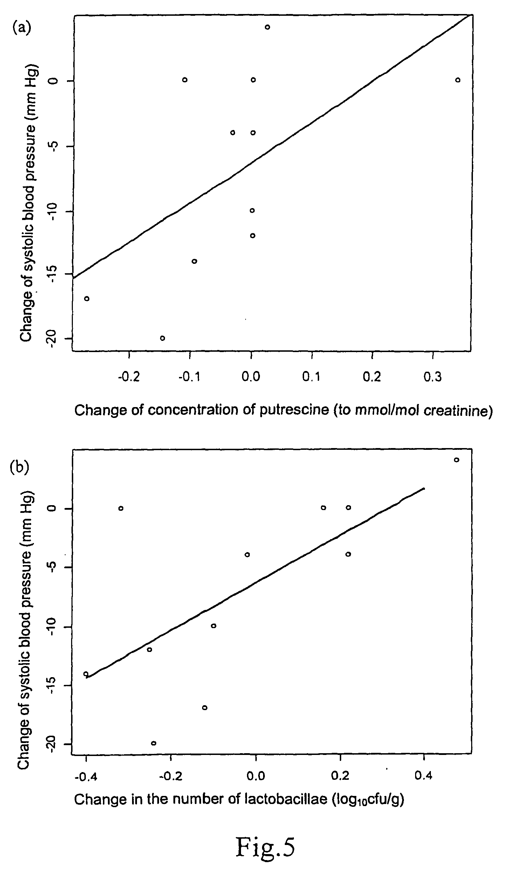

Fig.5. Reduction of systolic blood pressure (end of the trial in comparison with the values of systolic blood pressure at the recruitment)
(a) Positive correlation with the minimal increase of lactobacilli counts (FISH) after the consumption *of L. plantarum* Tensia DSM 21380 comprising cheese (r=0.615, p=0.044, n=11)
(b) correlation with the increase of putrescine in urine of volunteers after the consumption *of L. plantarum* Tensia DSM 21380 comprising cheese in comparison with the values at the recruitment (r=0.631, p=0.037, n=11).

### DESCRIPTION OF THE EMBODIMENTS

### Example 1. Suppression of NSLAB microbes in food product

### Test with Estonian cheese

**Method.** Microorganism *Lactobacillus plantarum* Tensia DSM 21380 was added to the cheese milk of Dairy Cooperative E-Piim, (inoculation dose 3x10⁸ CFU/vat) and the milk was renneted (25 min). The curds were cut (25 min), heated (34°C 15 min), dried (25 min), pressed, drained (1 h), salted in brine (12°C; 20% NaCl; pH 4,7) 20 h, drained and dried (8 h), backed into plastic and ripened at 12°C for at least 4 weeks.

**Table 8. Lactobacilli content in L. plantarum Tensia DSM 21380 comprising cheese at 28^{th} day of ripening.**

| | *L. plantarum* Tensia DSM21380 comprising cheese | Control cheese pH 5.1 | *L.plantarum* Tensia DSM 21380 comprising cheese (used in volunteer trial) | | | Control cheese | | |
|---|---|---|---|---|---|---|---|---|
| | | | Day 3. | Day 28. | 3 months | Day 3. | Day 28. | 3 months |
| *Lactobacillus sp* | 10⁹ | 9x10⁵ | 6x10⁵ | 5x10⁸ | 5x10⁷ | ND | 10⁸ | 10⁷ |
| *L. plantarum* | 10⁹ | 2x10³ | 6x10⁵ | 5x10⁸ | 5x10⁷ | ND | ND | ND |
| *L. casei* | ND | 8x10⁵ | ND | ND | ND | ND | ND | ND |
| *L. buchneri* | ND | 10⁵ | ND | ND | ND | ND | ND | ND |
| OHOL* | ND | ND | ND | ND | ND | ND | 2x10³ | ND |

| | | | | | | | | |
|---|---|---|---|---|---|---|---|---|
| OHOL - obligatively homofermentative lactobacilli ND - not detected | | | | | | | | |

The *L. plantarum* counts were 10 thousand times higher in *L. plantarum* Tensia DSM 21380 comprising cheese to the day 28 to in comparison with the control cheese. While in control cheese the homofermentative lactobacilli, *L. casei* and *L. buchneri* strains were present, then all aforementioned species were missing in *L. plantarum* Tensia DSM 21380 comprising cheese. Thus the strain *Lactobacillus plantarum* Tensia DSM 21380 possesses the ability to inhibit cheese NSLAB and suppress the cheese contaminating microbiota. The latter can be pathogens occurring in food product after preparation. Therefore *L. plantarum* Tensia DSM 21380 addition could help to lengthen the shelf-life of a food product.

**Detection of *Lactobacillus plantarum* Tensia DSM 21380 counts in cheese by DGGE Method**. Microbial DNA was isolated from cheese by QIAamp DNA Mini Kit (QIAGEN) and amplified with primers 968-GC-f (GGGAACGCGAAGAACCTTA-GC), 1401-r (CGGTGTGTACAAGACCC).

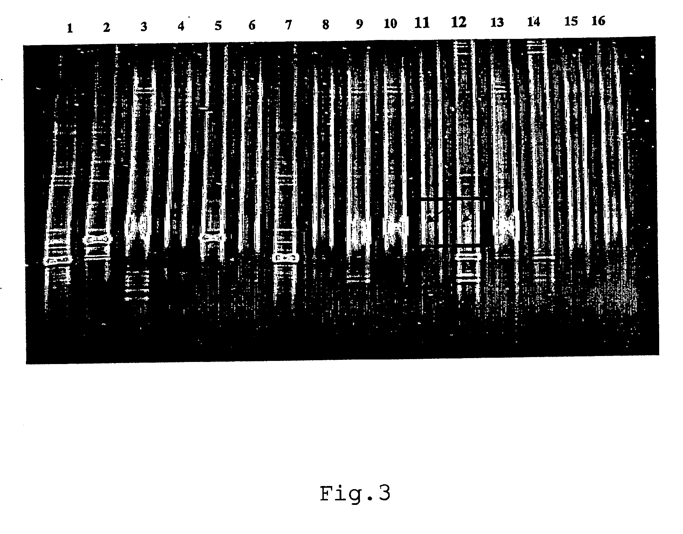

PCR products were separated by DGGE electrophoresis on a 30-60% acrylamide containing gel using Dcode™ System technique (Bio-Rad, Hercules, California) (Fig. 3).

**Detection of biogenic amines in *L. plantarum* Tensia DSM 21380 comprising cheese Method.** Cheese samples were extracted (20 ml 50% methanol solution was added to 10g of cheese and incubated at 45°C for 1 h, cooled to 30°C and centrifuged) and 200µl of upper layer was derivatized for GC analyze by modified method of Nakovichi (Nakovich, L. 2003 Analysis of biogenic amines by GC/FID and GC/MS) in Department of Microbiology of the University of Tartu.

GC analysis were carried out by gas chromatograph HP 6890 Series GC System, with capillary colonna HP-5 19091J-413 (30 m×0,32 mm; 0,25 µm). The column temperature program 160°C 1 min, 20°C/min 280°C 15 min; and detector (FID) 350°C.

**Table 9. Biogenic amines and polyamines in L. plantarum Tensia DSM 21380 comprising test-cheeses from industrial test trials**

| Sample | Viable counts of strain incorporated into cheese (CFU/g) at day 3-4 after preparation | Amines (mg/kg) | | | Viable count of *L*. *plantarum* Tensia DSM 21380 in ripe cheese |
|---|---|---|---|---|---|
| | | Tyramine | Putrescine | Cadaverine | |
| *L. plantarum* Tensia DSM 21380, 1. Batch | 6.5x10⁸ | 0.69 | 1.32 | 0 | 10⁶ |
| Control cheese 1. Batch | - | 2.31 | 1.82 | 0 | - |
| *L. plantarum* Tensia DSM 21380, 2. Batch | 4.5x10⁶ | 2.65 | 7.46 | 0 | 6x10⁸ |
| Control cheese 2. Batch | | 5.64 | 1.84 | 0 | 10⁷ |
| *L. µplantarum* Tensia DSM 21380, 3. Batch | 2x10⁶ | 5.49 | 7.29 | 0 | 10⁸ |
| Control cheese 3. Batch | - | 0 | 0 | 0 | - |

*L. plantarum* Tensia DSM 21380 produced tyramine 0.69-5.49 mg per kg of cheese and putrescine in lower quantities: 1.32-7.46 mg/kg.

### Example 2. Lactobacillus plantarum Tensia DSM 21380 safety trial with NIH mice

For the acceptance by the Estonian Veterinary and Food Board, the safety of probiotic strains and the food containing theses strains should be tested.

**Method.** In the experimental model with NIH mice 3 groups of mice consumed different cheeses during 30 days (control cheese with no additives, *Lactobacillus plantarum* Tensia DSM 21380 comprising cheese). Mice stayed in good condition, cheese administration caused increase of body weight, no changes in fur and digestion was detected. No translocation of administrated strains or other microbes into blood or organs was detected. No pathological shifts were found by the morphological and histological evaluation of the liver and spleen which proves the safety of *Lactobacillus plantarum* strain Tensia DSM 21380.

### Example 3. Effect of Lactobacillus plantarum Tensia DSM 21380 comprising cheese on blood indices and intestinal microflora of healthy volunteers

The aim of the clinical trial (randomized blinded cross-over placebo controlled) was to evaluate the safety and effect on intestinal microflora of cheese comprising antimicrobial *Lactobacillus plantarum* Tensia DSM 21380 on healthy volunteers.

**Persons and Methods:** Participants were healthy volunteers, both male and female 12 persons (M/F 5/7; 21-43 years). For exclusion criteria diabetes, glucose and glycohemoglobin HbA1c from blood sera were detected.

Test cheeses comprised strain *Lactobacillus plantarum* Tensia DSM 21380 (viable counts of 5x 10⁸ CFU/g cheese). Before consumption the test-cheese was incubated with *Lactobacillus plantarum* DSM 21380 Tensia for 30 days at 12°C. Regular Estonian cheese without additives served as a control. Trial was a randomized blinded cross-over placebo controlled trial. Trial started with 3-week consumption of test cheese, followed by 2 week washout period, after which the control cheese was consumed for 3 weeks. Dose 50 g /day.

**Table 10. Clinical data of healthy volunteers after consumption of probiotic cheese comprising L. plantarum Tensia DSM 21380**

| | Probiotic cheese with *L*. *plantarum* Tensia DSM 21380 | | Control cheese | | *P* values |
|---|---|---|---|---|---|
| | Baseline | After treatment | Baseline | After treatment | |
| BMI (kg/m²) | 24.1±3.6 | 24.2 ± 3.6 | 23.8 ± 3.5 | 23.9 ± 3.6 | 0.625/0.399 |
| Systolic pressure (mm Hg) | 112.9 ± 10.4 | 107.1 ± 10.4 | 110.3 ± 8.3 | 109.3 ± 9.4 | 0.016/0.655 |
| Diastolic pressure (mm Hg) | 78.8±7.1 | 74.3±8.8 | 74.8±5.2 | 75.7±8.3 | 0.021/0.668 |

| | | | | | |
|---|---|---|---|---|---|
| Body mass index (BMI) (kg/m²): 19-25 kg/m² - normal, 26-30 kg/m² - overweight, over 30 - obesity. | | | | | |

Both systolic and diastolic pressure were significantly lower after 3-week consumption of probiotic *L. plantarum* Tensia DSM 21380 (5x10⁸CFU/g x 50 g) comprising cheese. The 3-week consumption of strain Tensia DSM 21380 comprising cheese did not increase the body mass index.

**Effect of *Lactobacillus plantarum* Tensia DSM 21380 on intestinal microflora Method.** Quantitative analysis of fecal microflora and species of lactobacilli, methods elaborated in Department of Microbiology of Tartu University were (Mikelsaar ME, Väljaots ME, Lenzner AA. Anaerobe Inhalts- und Wandmikroflora des Magen-Darm-Kanals. Die Nahrung, 1984, 23, 6/7, 727-733; Sepp E., Julge K., Vasar M., Naaber P., Björksten B., Mikelsaar M. Intestinal microflora of Estonian and Swedish infants. Acta Paediatrica, 1997, 86, 956-961).

**Table 11. Lactobacilli counts in the faeces of healthy volunteers (log₁₀ cfu/g faeces)**

| | *L. plantarum* Tensia DSM 21380 comprising cheese | | Control cheese | | P values paired t-test BL1 vs PRO/ BL2 vs PL |
|---|---|---|---|---|---|
| | BL1 mean±stdev range (median) | PRO mean±stdev range (median) | BL2 mean±stdev range (median) | PL mean±stdev range (median) | |
| LB total count *cultivation* | 5.1 ± 1.9 0-6.3 (5.9) | 6.7 ± 1.0 4.9 - 8.6 (6.7) | 5.7 ± 1.4 4.0 - 8.6 (5.3) | ND | 0.025 / |
| LB total count *FISH* | 8.4 ± 0.1 8.1 - 8.6(8.4) | 8.4 ± 0.3 7.9 - 9.1(8.3) | 8.4 ± 0.3 8.0 - 8.8 (8.3) | 8.3 ± 0.3 7.6 - 9.0 (8.2) | 0.748 / 0.244 |
| *L. plantarum ** range/median prevalence | 0 - 5.3 (0) 3/12 | 0 - 8.6 (5.9) 10/12 § | 0 - 4.3 (0) 2/12 | ND | 0.006 /ND |

| | | | | | |
|---|---|---|---|---|---|
| ND - not determined | | | | | |

The total count of lactobacilli and the prevalence of L. *plantarum ** increased (p=0.006) in faeces of volunteers.

**Table 12. Prevalence of L. plantarum as species in fecal samples of volunteers at the recruitment (BL1), after probiotic cheese consumption (PRO) and recovery (BL 2)**

| Group | BL 1 | PRO | BL2 |
|---|---|---|---|
| Group 1 (n=12) | 3/12 | 10/12 | 2/12 |
| Group 2 (n=12) | 2/12 | 3/12 | 4/12 |

Increase of *L. plantarum* as species was detected *(Fischer exact test,* 3/12 vs 10/12, p=0.006) in the *L. plantarum* Tensia DSM 21380 group.

Thus, *L. plantarum* Tensia DSM 21380 affects positively human GIT microflora though increase of beneficial lactobacilli counts.

Surprisingly, by increasing the counts of facultative heterofermentative lactobacilli (FHEL; 4.7±1.3 vs. 5.7±1.3, p= 0.029), the fermentation group, where *L. plantarum* Tensia DSM 21380 belongs, the increase of obligatively homofermentative lactobacilli (OHOL) count was detected (4.6±1.1 vs. 5.7±1.5, p= 0.014). Thus, *L. plantarum* Tensia DSM 21380 helps significantly to stabilize GT lactoflora.

### Fecal lactobacilli counts of volunteers by molecular methods (fluorescent in situ hybridization, FISH)

**Method.** Fecal samples were diluted to 1/10 in PBS buffer. Microbial cells were fixed with 4% formaldehyde solution and kept at 4°C. FISH was carried out by method of Harmsen with the probe Lab 158 tagged with dye Cy 3. Tagged microbial cells were counted fluorescence microscopicy.

Results are presented in table 11 together with the results of bacteriological analyses. According to FISH, (the method that registers also dead cells) the lactobacilli counts remained practically unchanged.

### The lactobacilli DGGE profile from faeces of L. plantarum Tensia DSM 21380 comprising volunteers that consumed cheese.

**Method.** Subsequently, the PCR product was separated by DGGE electrophoresis in 30-60% acrylamide containing gel with Dcode™ System technique. (Bio-Rad, Hercules, California). Gels were analyzed by BioNumerics 2.5 (Applied Maths, Belgium) software according to Peasoni correlation (Heilig HG, Zoetendal EG, Vaughan EE, Marteau P, Akkermans, ADL, de Vos WM. Molecular diversity of Lactobacillus ssp. and other Lactic acid bacteria in the human intestine as determined by specific amplification of 16S ribosomal DNA. Appl Envir Microbiol 2002; 68: 114-123).

Gel photo (Fig. 4) indicates profile of dominant lactobacilli species in the subject's faeces according to 16S rDNA amplification. With the UMPAG software it was possible to analyze putative matrix coincidence between different gels, i.e. to detect significant difference (>20%) between different gels/different analysis.

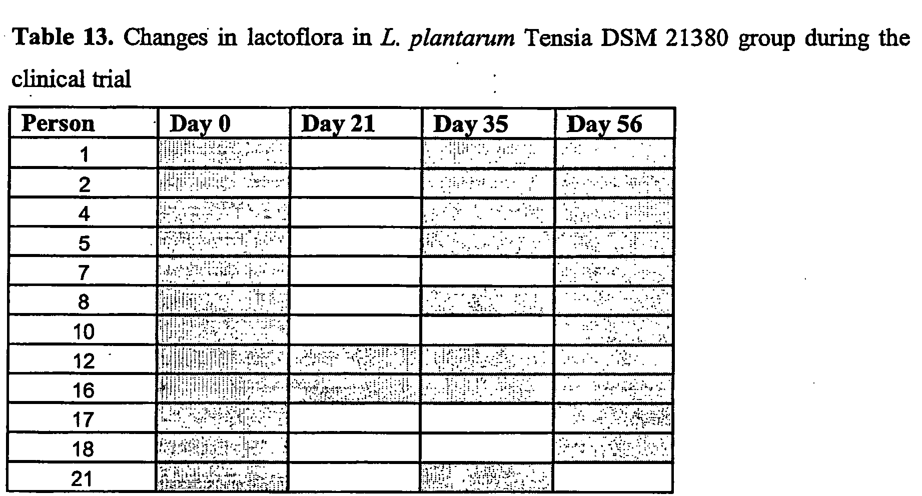

Table 13 indicates, that in the *L. plantarum* Tensia DSM 21380 group the indigenous lactoflora pattern changed in 10 persons from 12 to the day 21 (p<0.05).

According results were obtained in *L. plantarum* colonization survey by bacteriological method. The changes remained stabile in 4 persons even after 2 weeks.

### The polyamines and biogenic amines content in the urine of volunteers

For the evaluation of biogenic amines before and after *L. plantarum* Tensia DSM 21380 comprising cheese consumption and at the stabilization period, the morning urine and gas chromatography method were used.

**Method.** Urine samples were derivatized with propylchlorophormate for GC analyze by modified method of Ugland (Ugland HG; Krough M, Rasmussen KE: Aqueous alkylchloroformate derivatization and solid-phase microextraction: determination of amphetamines in urine by capillary gas chromatography. J Chromatography B Biomed Sci Appl 1997; 701:29-38).

GC analysis were carried out by gas chromatograph HP 6890 Series GC System (Hewlett Packard, Avondale, PA, USA), with capillar colonne HP-5 19091J-433 (30 m ×0.25 mm; 0.25 µm) The column temperature program 150°C 1 min, 20°C/min 280°C for 5 min; and detector (FID) 250°C. The biogenic amines concentration was calculated according to nmol / mol creatinine.

**Table 14. Polyamines in volunteers consuming L. plantarum Tensia DSM 21380 cheese assessed in morning urine (nmol/mol creatinine)**

| | Probiotic cheese with *L. plantarum* Tensia DSM 21380 additive | | Control cheese | | P values |
|---|---|---|---|---|---|
| | BL1 mean±stdev range (median) | PRO mean±stdev range (median) | BL2 mean±stdev range (median) | PL mean±stdev range (median) | *paired t-test* BL1 vs PRO / BL2 vs PL |
| Put | 0.110 ± 0.139 0 - 0.467 (0.046) | 0.090 ± 0.120 0-0.396 (0.041) | 0.073 ± 0.110 0 - 0.384 (0.037) | 0.027 ± 0.024 0- 0.055 (0.037) | 0.496/0.275 |
| acPut | 0.607 ± 0.558 0 - 2.159 (0.478) | 0.567 ± 0.431 0.191 - 1.758 (0.430) | 0.817 ± 1.027 0.091 - 3.916 (0.550) | 0.573 ± 0.391 0.182 - 1.403 (0.486) | 0.510/0.677 |
| DAP | 0.148 ± 0.125 0 - 0.344 (0.159) | 0.056 ± 0.084 0 - 0.205 (0) | 0.090 ± 0.163 0 - 0.432 (0) | 0.061 ± 0.099 0 - 0.246 (0) | 0.016/0.844 |
| acSpd | 0.181 ± 0.137 0 - 0.428 (0.144) | 0.227 ± 0.142 0.053 - 0.493 (0.182) | 0.197 ± 0.148 0.039 - 0.495 .195) | 0.258 ± 0.221 0.041 - 0.686 (0.186) | 0.016/0.244 |
| Cad | 0.012 ± 0.026 0 - 0.084 (0) | 0.038 ± 0.062 0 - 0.156 (0) | 0.041 ± 0.077 0 - 0.248 (0) | 0.015 ± 0.037 0 - 0.123 (0) | 0.125/0. |

| | | | | | |
|---|---|---|---|---|---|
| BL1- baseline 1, at the recruitment, PRO -after probiotic treatment, BL 2-baseline 2, recovery Put - putrescine, acPut - N-acetylputrescine, DAP - 1.3-diaminopropane, acSpd - N8-acetylspermidine, Cad - cadaverine | | | | | |

Consumption of *L. plantarum* Tensia DSM 21380 comprising probiotic cheese increased the acetylated spermidine in urine of volunteers at the end of probiotic period indicating the improvement of the metabolism of polyamines in blood and tissues. DAP concentration in urine decreased.

### Correlations

It was found that the reduction of systolic blood pressure was correlated to the increase of Lactobacilli counts and the increase of putrescine in urine (Fig.5).

### SAFETY

After cheese trial with volunteers, the values of systemic inflammation markers (U-CRP, ultrasensitive CRP, and leucocytes) were not changed and were within the normal range (Table 15). No change was detected also in the values of essential allergy marker IgE. The consumption of probiotic cheese did not cause changes in WBC counts (leucogram): the proportion of cells at the end of the trial remained unchanged in comparison with the recruitment values.

**Table 15. Inflammation markers at the beginning and the end of the trial**

| | Probiotic cheese with *L. plantarum* Tensia DSM 21380 | | Control cheese | | P values | Standard values |
|---|---|---|---|---|---|---|
| | before | after | before | after | | |
| U-CRP, mg/L | 1.1 ± 0.6 | 1.0 ± 0.3 | 1.4 ± 0.9 | 1.6 ± 1.3 | 0.3/0.9 | <5mg/L |
| Leucocytes total count x10⁹/L | 5.2 ± 0.8 | 5.6 ± 1.3 | 5.1 ± 1.1 | 5.5 ± 1.1 | 0.6/0.14 | 4 - 10 x 10⁹/L |

Consumption of *L. plantarum* Tensia DSM 21380 comprising probiotic cheese did not cause abdominal discomfort (abdominal pain, flatulence, bloating) or the increase of the body mass index, glucose and lipid levels.

Thus in healthy subjects the consumption of *Lactobacillus plantarum* Tensia DSM 21380 comprising cheese does not cause systemic inflammation, allergic sensibilisation nor does it cause harm to essential organs.

### Example 4. Effect of Lactobacillus plantarum DSM 21380 comprising cheese on blood indices of elderly healthy individuals

During the clinical trial (randomized blinded cross-over placebo controlled) the safety and effect on blood indices of *Lactobacillus plantarum* Tensia DSM 21380 comprising cheese on elderly healthy volunteers was evaluated.

**Persons and Methods**. Participants were healthy volunteers, both male and female 21 persons (M/F 2/19; 61-84 years). For exclusion criteria diabetes, glucose and glycohemoglobin HbA1c from blood sera were detected.

Test cheeses comprised strain *Lactobacillus plantarum* Tensia DSM 21380 (viable counts of 2x 10⁷ CFU/g cheese). Before consumption the test-cheese was incubated with *Lactobacillus plantarum* Tensia DSM 21380 for 30 days at 12°C. Regular Estonian cheese without additives served as a control. Trial was a randomized blinded cross-over placebo controlled trial. Trial started with 3-week consumption of test -cheese, followed by 2 week washout period, after which the control-cheese was consumed for 3weeks. Dose 50 g /day.

**Table 16. Clinical data of elderly healthy volunteers after consumption of probiotic L. plantarum Tensia DSM 21380 comprising cheese**

| | Probiotic cheese with *L*. *plantarum* Tensia DSM 21380 | | Control cheese | | *P* values |
|---|---|---|---|---|---|
| | Baseline | After treatment | Baseline | After treatment | |
| BMI (kg/m²) | 27.6 ± 4.1 | 27.5 ± 4.2 | 27.5 ± 4.0 | 27.6 ± 4.2 | 0.723/0.793 |
| Systolic pressure (mm Hg) | 138.1 ± 16.6 | 132.2 ± 16.2 | 138.7 ± 21.4 | 135.2 ± 21.1 | **0.038**/0.185 |
| Diastolic pressure (mm Hg) | 77.2 ± 7.7 | 73.1 ± 8.0 | 76.4 ± 8.9 | 74.8 ± 9.2 | **0.004** /0.246 |

| | | | | | |
|---|---|---|---|---|---|
| Body mass index (BMI) (kg/m²): 19-25 kg/m² - normal, 26-30 kg/m² - overweight, over 30 - obesity. | | | | | |

The decrease of blood pressure (both systolic and diastolic) was detected on elderly subjects after the 3-week consumption of probiotic *L. plantarum* Tensia DSM 21380 (2x 10⁷/g x 50 g) comprising cheese. At the same time the consumption of cheese of relatively high fat content did not increase the body mass index of elderly volunteers (Table 16).

### SAFETY

The consumption of *L. plantarum* DSM 21380 comprising probiotic cheese by elderly did not cause abdominal discomfort (abdominal pain, flatulence, bloating).

After the cheese trial the values of systemic inflammation markers (U-CRP, and leucocytes) of the participants were not increased and were within the normal range (Table 17).

**Table 17. The inflammation markers of blood of the elderly healthy volunteers at the recruitment and at the end of the trial**

| | Probiotic *L. plantarum* Tensia DSM 21380 comprising cheese | | Control cheese | | P values | Standard values |
|---|---|---|---|---|---|---|
| | before | after | before | after | | |
| U-CRP, mg/L | 0.13/0.81 | 0.13/0.81 | 0.13/0.8 | 0.13/0.81 | 0.13 /0.81 | < 5 mg/L |
| Leucocytes total count x10⁹/L | 5.1 ± 1.3 | 4.9 ± 1.3 | 5.0 ± 1.0 | 5.0 ± 1.6 | 0.33 / 0.33 | 4 - 10 x 10⁹/L |

No abnormalities were detected also in the values of essential allergy marker IgE or the kidney and liver markers (serum creatinine, albumine, alanine transaminase (ALAT), aspartate transaminase (ASAT)).

Comparing the total cholesterol levels of the treatment and placebo period, significant decrease was detected after the consumption of the *L*. *plantarum* DSM 21380 comprising cheese. The raise of the blood serum glucose content by 0.3 units after the probiotic cheese consumption was an undesirable effect, but in comparison with the placebo period the change was not statistically significant (Table 18) and remained within the normal range.

**Table 18. Blood serum biochemical parameters of elderly healthy volunteers after consumption of L. plantarum DSM 21380 comprising cheese.**

| | Probiotic cheese | | Control cheese | | *P* values (BL1 vs BL2, PRO vs PL) |
|---|---|---|---|---|---|
| | BL1 | PRO | BL2 | PL | |
| Glycose, mmol/l | 5.1 ± 0.5 | 5.4 ± 0.5 | 5.3 ± 0.5 | 5.4 ± 0.4 | **0.036**/0.144 (0.212, 0.757) |
| Total cholesterol, mmol/l | 5.7 ± 0.8 | 5.6 ± 0.8 | 5.9 ± 0.9 | 5.7 ± 0.8 | 0.343/0.198 (**0.044**, 0.087) |
| HDL- cholesterol, mmol/l | 1.7 ± 0.4 | 1.6 ± 0.4 | 1.7 ± 0.4 | 1.7 ± 0.5 | 0.411/0.514 (0.073, 0.118) |
| LDL- cholesterol, mmol/l | 3.9 ± 0.8 | 3.8 ± 0.7 | 4.1 ± 0.9 | 3.8 ± 0.7 | 0.557/0.052 (0.075, 0.9187) |
| Triglycerides, mmol/l | 1.1 ± 0.6 | 1.1 ± 0.6 | 1.2 ± 0.5 | 1.1 ± 0.5 | 0.380 /0.394 (0.411, 0.097) |

Thus in elderly healthy individuals the consumption of *Lactobacillus plantarum* Tensia DSM 21380 comprising cheese does not cause systemic inflammation, allergic sensibilisation nor cause harm to essential organs.

## Claims

1. Isolated microorganism strain *Lactobacillus plantarum* Tensia DSM 21380 as antimicrobial and antihypertensive probiotic that produces conjugated linoleic acid (CLA), hydrogen peroxide (H₂O₂), nitric mono-oxide (NO) and possesses antioxidatitvity and plantaricin encoding genes plnE, plnF, plnJ, plnK in order to
- suppress contaminating microbes in food products,
- dominate within colonic lactobacilli,
- increase the metabolism of polyamines in organism,
- lower blood pressure.

2. Microorganism strain according to claim 1, **characterized in that** the named microbes are nonstarter lactobacilli and/or pathogens originated from raw milk and/or pathogens occasionally contaminating food after preparation.

3. Microorganism strain according to any of claims 1-2 in freeze-dried form.

4. A food product comprising the microorganism strain according to any of claims 1-3.

5. The food product according to claim 4, wherein the food product is a dairy product.

6. The food product according to claim 5, wherein the food product is a fermented milk product

7. The food product according to claim 6, wherein the food product is cheese.

8. Composition comprising the microorganism strain according to any of claims 1-3.

9. Use of microorganism according to any of claims 1-3 for preparation of blood pressure lowering medicine.

10. A method for suppressing pathogens and nonstarter lactobacilli in food product comprising adding of the culture of the microorganism or freeze-dried powder of microorganism according to any of claims 1-3, to the food product

11. The method according to claim 10, wherein the food product is a dairy product.

12. The method according to claim 11, wherein the dairy product is a fermented milk product, cheese, cottage cheese, curds, yoghurt, ice-cream, butter, spread cheese.

13. Method for the extension of the shelf-life of the food product comprising adding the microorganism according to any of claims 1-3 to the food product.

## Patentansprüche

1. Isolierter Mikroorganismenstamm *Lactobacillus plantarum* Tensia DSM 21380 als antimikrobielles und blutdrucksenkendes probiotisches Nahrungsmittelprodukt, das konjugierte Linolsäure (CLA), Wasserstoffperoxid (H₂O₂), Stickstoffmonooxid (NO) erzeugt und antioxidantische Wirkung hat, sowie für die Erzeugung von Plantarizin erforderliche Gene plnE, plnF, plnJ, plnK besitzt, um
- kontaminierende Mikroorganismen in Nahrungsmittelprodukten zu supprimieren,
- unter Lactobacilli im Darm zu dominieren,
- Metabolismus von Polyaminen im Organismus zu vergrößern,
- Blutdruck zu senken.

2. Mikroorganismenstamm nach Anspruch 1, **dadurch gekennzeichnet, dass** die genannten Mikrobe von Rohmilchstammende Nicht-Starter-Lactobacilli und/oder Pathogene sind, und/oder kontaminierende Pathogene , welche nach der Nahrungsherstellung gelegentlich entstanden sind.

3. Mikroorganismenstamm nach einem der vorangehenden Ansprüche 1-2 in kaltgetrockneter Form.

4. Nahrungsprodukt welches Mikroorganismenstamm nach einem der vorangehenden Ansprüche 1-3 enthält.

5. Nahrungsprodukt nach Anspruch 4, wobei dieses Nahrungsprodukt ein Milchprodukt ist.

6. Nahrungsprodukt nach Anspruch 5, wobei dieses Nahrungsprodukt ein fermentiertes Milchprodukt ist.

7. Nahrungsprodukt nach Anspruch 6, wobei dieses Nahrungsprodukt Käse ist.

8. Zusammensetzung umfassend den Mikroorganismus nach einem der vorangehenden Ansprüche 1-3.

9. Verwendung des Mikroorganismus nach einem der vorangehenden Ansprüche 1-3 für die Herstellung von blutdrucksenkenden Arzneimitteln.

10. Verfahren zum Supprimieren von Pathogenen und Nicht-Starter-Lactobacilli in einem Nahrungsprodukt durch Zufügung einer Kultur vom Mikroorganismus oder des kaltgetrockneten Mikroorganismus nach einem der vorangehenden Ansprüche 1-3 zum Nahrungsproduct.

11. Verfahren nach Anspruch 10, wobei dieses Nahrungsproduct ein Milchprodukt ist.

12. Verfahren nach Anspruch 11, wobei dieses Nahrungsprodukt ein kaltgetrocknetes Milchprodukt, Käse, Hüttenkäse, Quark, Joghurt, Eiscreme, Butter, Streichkäse ist.

13. Verfahren zur Verlängerung von der Haltbarkeitsdauer des Nahrungsprodukts durch Zufügung des Mikroorganismus nach einer der vorangehenden Ansprüche 1-3 zum Nahrungsprodukt.

## Revendications

1. La souche de microorganisme isolé *Lactobacillus plantarum Tensia* DSM 21380 comme probiotique antimicrobienne et antihypertensif, produisant d'acide linoléique conjugué (CLA), de le peroxyde d'hydrogène (H₂O₂) de l'oxyde nitrique (NO) et possède des propriétés antioxydantes et des gènes plnE, plnF, plnJ, plnK codant des plantaricines en ordre de :
- réprimer la contamination des microbes dans les produits alimentaires,
- à dominer dans les lactobacilles colique
- augmenter le métabolisme des polyamines dans le corps,
- baisser la pression artérielle.

2. La souche de microorganisme selon la revendication 1, **caractérisé en ce que** les microbes susnommés sont lactobacilles non utilisés comme levain et / ou des pathogènes provenant de lait cru et / ou des pathogènes ayant contaminé la nourriture après la préparation de façon occasionnelle.

3. La souche de microorganisme selon l'une quelconque des revendications 1 à 2, dans une forme lyophilisée.

4. Le produit alimentaire comprenant la souche de microorganisme selon l'une quelconque des revendications 1 à 3.

5. Le produit alimentaire selon la revendication 4, dans lequel le produit alimentaire est un produit laitier.

6. Le produit alimentaire selon la revendication 5, dans lequel le produit alimentaire est un produit laitier fermenté.

7. Le produit alimentaire selon la revendication 6, dans lequel le produit alimentaire est un fromage.

8. La composition comprenant la souche de microorganisme selon l'une quelconque des revendications 1 à 3.

9. L'utilisation de microorganisme selon l'une quelconque des revendications 1 à 3 pour la préparation des médecines antihypertenseurs.

10. Le procédé pour supprimer les agents pathogènes et les lactobacilles non utilisés comme levain dans le produit alimentaire, comprenant l'addition de la culture du micro-organisme ou de la poudre lyophilisée de micro-organisme au produit alimentaire selon l'une quelconque des revendications 1 à 3.

11. Le procédé selon la revendication 10, dans lequel le produit alimentaire est un produit laitier.

12. Le procédé selon la revendication 11, dans lequel le produit laitier est un produit laitier fermenté, fromage, caillés, lait caillé, yaourt, crème glacée, beurre, fromage à tartiner.

13. Le procédé pour l'extension de la durée de conservation du produit alimentaire, comprenant l'addition du micro-organisme pour au produit alimentaire selon l'une quelconque des revendications 1 à 3.
